# EUROPÄISCHE PATENTSCHRIFT

(11) **EP 1 841 798 B1**
(45) Veröffentlichungstag und Bekanntmachung des Hinweises auf die Patenterteilung: **25.11.2020**
(21) Anmeldenummer: 06701005.8
(22) Anmeldetag: 16.01.2006
(51) Int. Cl.: C08F 6/00, C08F 220/56, C08F 2/48

(54) **WASSERLÖSLICHE ODER WASSERQUELLBARE POLYMERISATE, INSBESONDERE WASSERLÖSLICHE ODER WASSERQUELLBARE COPOLYMERISATE AUS ACRYLAMID UND MINDESTENS EINEM IONISCHEN COMONOMEREN MIT NIEDRIGEM RESTMONOMERENGEHALT**
WATER-SOLUBLE OR WATER-SWELLABLE POLYMERS, PARTICULARLY WATER-SOLUBLE OR WATER-SWELLABLE COPOLYMERS MADE OF ACRYLAMIDE AND AT LEAST ONE IONIC COMONOMER HAVING A LOW RESIDUAL MONOMER CONCENTRATION
POLYMERES SOLUBLES DANS L'EAU OU POUVANT GONFLER DANS L'EAU, EN PARTICULIER COPOLYMERES CONSTITUES D'AMIDE ACRYLIQUE ET D'AU MOINS UN COMONOMERE IONIQUE A FAIBLE TENEUR EN MONOMERES RESIDUELS

(30) Priorität: 28.01.2005 DE 102005004285
(43) Veröffentlichungstag der Anmeldung: 10.10.2007
(73) Patentinhaber: Evonik Operations GmbH, 45128 Essen (DE)
(72) Erfinder: HERTH, Gregor, 83308 Trostberg (DE); DANNEHL, Manfred, 63796 Alzenau (DE); STEINER, Norbert, 63755 Alzenau (DE)
(74) Vertreter: Evonik Patent Association
(86) Internationale Anmeldenummer: PCT/EP2006/000328
(87) Internationale Veröffentlichungsnummer: WO 2006/079462

(56) Entgegenhaltungen:
- JP-A- S63 295 604
- US-A1- 2004 077 744
- US-A1- 2004 077 744
- PATENT ABSTRACTS OF JAPAN Bd. 013, Nr. 124 (C-580), 27. März 1989 (1989-03-27) & JP 63 295604 A (MITSUBISHI RAYON CO LTD; others: 01), 2. Dezember 1988 (1988-12-02)

## Beschreibung

Die Erfindung betrifft ein Verfahren zur Herstellung wasserlöslicher oder wasserquell-barer Polymerisate mit niedrigerem Restmonomerengehalt, insbesondere auf Basis von Acrylsäure und/oder Acrylamid und mindestens einem weiteren wasserlöslichen Comonomeren.

Wasserlösliche oder wasserquellbare Copolymere werden heute in einer Vielzahl von Bereichen industriell eingesetzt. In wasserlöslicher Form erfolgt ihr Einsatz beispielsweise als Flockungshilfsmittel zur Klärung kommunaler und industrieller Abwässer, als Bohrspülmittel in der Erdölförderung, als Bodenverbesserungsmittel, Verdickungsmittel, Dispergierhilfsmittel und Retentionshilfsmittel etc.

Das Einsatzgebiet der entsprechenden vernetzten wasserquellbaren Polymerisate ist ebenfalls sehr vielfältig. So finden sie beispielsweise Verwendung in der Personal Care Industrie bei der Herstellung von Hygieneartikeln, wie z.B. absorbierenden Sanitärartikeln für die Kinder- und Erwachsenhygiene in Form von Babywindeln, Damenbinden, Tampons, Inkontinenzartikeln sowie Produkten zur Wundabdeckung, in die sie eingearbeitet sind.

In diesen Produkten werden die charakteristischen Absorptionseigenschaften dieser wasserquellbaren Polymerisate genutzt, weil sie ein Vielfaches ihres Gewichtes an wäßrigen Flüssigkeiten oder Körperflüssigkeiten, wie z.B. Blut oder Urin unter Quellung und Ausbildung von Hydrogelen aufnehmen und dieses Flüssigkeitsvolumen selbst bei Einwirkung eines äußeren Druckes zurückhalten können. Diese Eigenschaften macht man sich auch in der Verpackungsindustrie zunutze, in dem Nonwoven-Produkte, in die diese wasserquellbaren Polymerisate eingearbeitet sind, als Verpackungskomponente, insbesondere in Form von Saugeinlagen für Fisch- und Fleischschalen verwendet werden.

Weitere Anwendungsgebiete solcher wasserquellbaren Polymerisate, die üblicherweise aus wässriger Lösung hergestellt werden, wobei sich während der Polymerisation ein festes Gel bildet, das im Rahmen der Aufarbeitung zu einem granularen Endprodukt verarbeitet wird, sind die Agrartechnik bzw. Land- und Gartenwirtschaft. Dort werden wasserquellbare Polymerisate u.a. als Bodenhilfsstoffe zur Wasser- und Nährstoffspeicherung, künstlicher Boden zur Pflanzenzüchtung sowie als Wurzelschutzgele verwendet. In der Kabelindustrie bzw. Nachrichtentechnik werden solche wasserquellbaren Polymerisate als flüssigkeitsabsorbierende und/oder flüssigkeitsabdichtende Komponenten bzw. als Additive in strom- oder lichtleitenden Kabeln eingesetzt.

Insbesondere der wachsende Einsatz von vernetzten wasserquellbaren Polymerisaten auf Acrylsäurebasis als sogenannte Superabsorber in Hygieneprodukten führte zu einer intensiven Forschungs- und Entwicklungstätigkeit, die sich im Umfang der diesbezüglichen Patentliteratur widerspiegelt und die auch eine direkte Folge der gesteigerten Anforderungen der Personal Care Industrie an das Eigenschaftsprofil dieser Polymerisate ist.

Nachdem anfänglich flüssigkeitsabsorbierende wasserquellbare Polymerisate mit sehr hohem Quellvermögen bzw. freier Quellkapazität bei Kontakt mit Flüssigkeit bevorzugt bei der Windelherstellung verwendet wurden, führten mechanistische Belastungsstudien an Windeln zu der Erkenntnis, daß durch die Bewegungen des Windelträgers verursachten Belastungen ein anderes Eigenschaftsprofil dieser Absorptionspolymerisate zur Herstellung von Windeln benötigt wird. Damit bei der Verwendung derartiger Superabsorber in einer Windelkonstruktion Flüssigkeitsaufnahme, Flüssigkeitstransport, Trockenheit der Windel und der Haut gewährleistet sind, ist ein ausgewogenes Verhältnis zwischen Absorptionsvermögen des Polymerisates (Gelvolumen) und Gelstärke des sich bei Flüssigkeitsaufnahme bildenden Hydrogels verbunden mit einem hohen Aufnahmevermögen unter Druckbelastung erforderlich.

Um superabsorbierende Polymerisate mit dem gewünschten Eigenschaftsprofil von hoher Retentionskapazität, hoher Gelstärke und hohem Aufnahmevermögen unter Druck zu erhalten, müssen die Oberflächen dieser absorbierenden Polymerisate nachträglich behandelt werden. Die nachträgliche Oberflächenbehandlung ist in der Patentliteratur vielfach beschrieben worden:
So werden in der US 4 043 952 zur Verbesserung der Dispergierbarkeit in Wasser polyvalente Metallverbindungen und in der US 4 051 086 zur Verbesserung der Aufnahmegeschwindigkeit Glyoxal empfohlen.

Die DE-OS-27 40 169 beschreibt die Herstellung von Absorptionsmitteln auf der Basis von kalium- und ammoniumacrylathaltigen Polymerisaten, die mit Polyolen behandelt und als Pulver und Folien in Windeln und anderen Hygieneprodukten sowie medizinischen Artikeln eingesetzt werden.

In den Patentschriften EP 0 083 022, DE-OS-33 31 644, DE-OS-35 07 755, DE-OS-35 23 617, DE-OS-36 28 482 und EP 0 349 240 wird die Nachbehandlung von Harzen mit zwei- oder mehrfunktionelle Gruppen enthaltenden Vernetzungsmitteln beschrieben, die mit den Carboxyl- oder Carboxylatgruppen oder anderen im Polymer enthaltenden Gruppen reagieren können. Hierbei wird entweder das Pulver direkt mit den Komponenten, ggf. unter Mitverwendung geringer Mengen Wasser und Lösungsmittel, vermischt oder das Pulver in einem inerten Lösungsmittel dispergiert oder 10 bis 40 Gew.-% Wasser enthaltende Polymere werden in einem hydrophilen oder hydrophoben Lösungsmittel dispergiert und anschließend oder gleichzeitig mit dem Vernetzungsmittel vermischt. Als Vernetzungsmittel können Polyglycidylether, Haloepoxiverbindungen, Polyole, Polyamine oder Polyisocyanate verwendet werden.

Neben diesen werden in DE-OS-33 14 019, DE-OS-37 37 196 weiterhin polyfunktionelle Aziridinverbindungen, Alkyl-di-(tri)-halogenide und öllösliche Polyepoxiverbindungen erwähnt.

In der DE-OS-35 03 458 erfolgt die Aufbringung eines Vernetzungsmittels auf ein Polymer in Gegenwart eines inerten anorganischen Pulvers wie SiO₂ ohne Verwendung organischer Lösungsmittel.

Nach der DE 40 20 780 C1 wird die verbesserte Absorption unter Druck durch die oberflächenvernetzende Behandlung eines Polymerharzes mit 0,1 bis 5 Gew.-% eines Alkylencarbonates erreicht.

Allen vorgenannten Verfahren ist gemeinsam, daß nach Auftragen des Oberflächenvernetzungsmittels eine Temperaturbehandlung der Polymerisate erfolgt.

Im Hinblick auf die vornehmliche Anwendung solcher vernetzter Absorberpolymerisate im Hygienebereich muß in besonderer Weise dem Umstand Rechnung getragen werden, daß die für den Einsatz in Babywindeln oder anderen Hygieneartikeln bestimmten Polymerisate keine hautsensibilisierende Wirkung beim Verwender dieser Produkte hervorrufen. Insbesondere muß gewährleistet sein, daß die beim Herstellungsprozeß verwendeten Ausgangsstoffe und Vernetzungsmittel so umgesetzt werden, daß ein gesundheitsgefährdendes Potential des Endproduktes ausgeschlossen werden kann, weil das Endprodukt in toxikologischer Hinsicht als unbedenklich anzusehen ist, d.h. die Polymerisate dürfen nur keinen oder lediglich einen sehr geringen Gehalt an Restmonomeren, deren Toxizität experimentell gut erforscht worden ist, aufweisen.

Dies gilt jedoch nicht nur für Polymerisate, die für den Einsatz in Hygieneprodukten bestimmt sind, sondern auch für wasserlösliche und wasserquellbare Polymerisate auf Basis von Acrylamid. So ist das toxische Potential von Acrylamid seit langer Zeit bekannt bzw. eine Vielzahl von Patenten und Publikationen befassen sich mit der Reduzierung des Acrylamids in solchen Polymerprodukten. Ein Weg, die Reduzierung des Restmonomerengehaltes von Acrylamid zu erreichen, basiert darauf, daß man nach der Polymerisation ein Additiv zusetzt, das sich an die Doppelbindung der Acrylamid-Restmonomeren addiert und diese damit umsetzt.

Es hat sich jedoch gezeigt, daß bei der Herstellung wasserlöslicher oder wasserquellbarer Polymerisate, eine vollständige Umwandlung der Monomeren - vor allem der Monomeren auf Acrylsäurebasis und auch auf Acrylamidbasis - nicht möglich ist. So ist festgestellt worden, daß die Herstellung eines vernetzten wasserquellbaren Polymerisates mit den gewünschten Eigenschaften von hoher Absorptionsgeschwindigkeit, hohem Retentionsvermögen und hoher Aufnahme bei Druckbelastung, verbunden mit einem möglichst geringen Restmonomerengehalt u.a. von der Vernetzerkonzentration und den eingesetzten Polymerisationskatalysatoren abhängt:
Ein hohes Retentionsvermögen wird bei einem schwach vernetzten Polymerisat erreicht. Demgegenüber erfordert eine hohe Aufnahme unter Druck ein stark vernetztes und hierdurch stabilisiertes Polymerisat, das erst aufgrund seiner Stabilität gegen einen äußeren Druck anquellen kann. Mit zunehmender Vernetzung steigt jedoch gleichzeitig auch der Restmonomerengehalt an, weil durch die schnell ansteigende Viskosität bei der Herstellung des Polymerisates sich die Diffusion der restlichen Monomermoleküle zu den reaktiven Radikalzentren verringert. Darüber hinaus bewirken restmonomerabsenkende Katalysatorsysteme oder erhöhte Katalysatorsysteme oft eine unerwünschte Abnahme der Retention.
Aufgrund der dargelegten Schwierigkeiten, ein Polymerisationsverfahren so zu führen, daß praktisch keine Restmonomeren entstehen und durch die für das Endprodukt geforderte toxikologische Unbedenklichkeit sind verschiedene Verfahren vorgeschlagen worden, in denen die Restmonomeren durch Umwandlung in ihre unbedenklichen Derivate aus den zuvor hergestellten Polymerisaten entfernt werden.

Beispielsweise wird in der DE-AS- 1 070 377 und US 2,960,486 die Behandlung des nach der Polymerisation erhaltenen hochmolekularen Polymergeles mit wässriger Natriumbisulfit- oder Natriummetabisulfit-Lösung und deren Trocknung bei 80 bis 120°C beschrieben. Dabei wurden Restacrylamidgehalte von 0,01% erhalten. Diese Verfahren setzten sich wegen ihrer Unwirtschaftlichkeit in der Praxis nicht durch, weil sie eine Verarbeitung der Polymerisate in sehr verdünnter Polymerlösung voraussetzen (2 bis 3 %ig).

In JP-PS 56/103207 wurden ebenfalls Bisulfite, Sulfite und Pyrosulfite zur Restmonomerenreduktion verwendet. Gasförmiges Schwefeldioxid wurde in der US 3,780,006 zur Herabsetzung der Acrylamidkonzentration in einem Emulsionspolymerisat benutzt.

Die US 3,755,280 beschreibt die Behandlung von Polymergel mit wäßriger Lösung von Natriumbisulfit oder-metabisufit bzw. in der EP 175 554 wird die Behandlung mit einem festen Alkalisulfit beschrieben. Hierdurch wurden Restmonomerengehalte von 0,03 bis 0,3 Gew.-% erhalten.

Die EP 0 505 163 beschreibt die Nachbehandlung von vernetzten Acrylsäurepolymerisaten mit einer Kombination von Metabisulfit und einer oberflächenaktiven Substanz (HLB von 3 bis 40), wodurch die Restmonomerengehalte bis zu 10 ppm gesenkt wurden. Für diese Nachbehandlung der Polymerisate wurden 2 bis 5 Gew.-% (bezogen auf das Polymergel mit 40% wS, d.h. 5 bis 12,5 Gew.-% Metabisulfit, bezogen auf das trockene Polymerisat) benötigt, um die genannte Restmonomerenabsenkung zu erreichen. Solch hohe Mengen an Zusatzstoffen können sich nachteilig auf die anwendungstechnischen Eigenschaften der Polymerisate auswirken.

WO 94/20547 beschreibt Zusätze wie Bromate und Chlorate zur Polymerisatlösung und eine nachfolgende Erhitzung des fertigen Polymers, bei dem durch Zusätze u.a. eine Reduzierung des Restmonomers erfolgt. Die Zugabe der Bromate und Chlorate kann auch nach der Polymerisation erfolgen. Trotz dieser Maßnahmen liegt der Restmonomerengehalt der Polymerisate zwischen etwa 135 und 1100 ppm.

EP 0 303 518 A2 beschreibt ein kontinuierliches bzw. diskontinuierliches Verfahren zur Herstellung von vernetzten Polyacrylatharzen, in dem Acrylsäure mit einem Neutralisationsgrad von 70 bis 100 Mol-% und ein wassermischbares oder wasserlösliches Polyvinylmonomer in wäßriger Lösung polymerisiert wird. Die zu polymerisierende wäßrige Monomerlösung weist eine Monomerkonzentration von mindestens 50% auf und zur Monomerenreduktion wird eine Kombination von einem thermischen Polymerisationsinitiator und einem Redoxinitiator eingesetzt. Die Polymerisation wird dabei so geführt, daß die bei der Polymerisation entstehende exotherme Reaktionswärme im wesentlichen die alleinige Wärmeenergie ist, die zur Durchführung der Polymerisation, der Vernetzung und der Verdampfung des eingesetzten Wassers verwendet wird, so daß eine nachfolgende Trocknung unterbleiben kann. Die auf diese Weise erhaltenen Polymerisate sollen im allgemeinen Restmonomerengehalte unter 500 ppm, bevorzugt unter 200 ppm aufweisen.

Die vorstehend genannten Verfahren ermöglichen zwar die Herstellung von Polymerisaten mit vermindertem Restmonomerengehalt. Jedoch erfordern die Verfahren vielfach eine komplizierte Reaktionsführung, die die Herstellungskosten der Polymerisate nicht unbeträchtlich verteuern. Insbesondere die Nachbehandlung mit schwefelhaltigen Verbindungen, die zum Teil als Zusatzstoff im Endprodukt verbleiben, ist als nachteilig anzusehen. Nicht nur das eine solche Nachbehandlung als zusätzlicher Reaktionsschritt die Verwendung weiterer Apparaturen, Geräte etc. beinhaltet, d.h. mit weiterem Zeitaufwand und somit Kosten verbunden sind. Es kann auch nicht ausgeschlossen werden, daß hierbei Oxidationsprodukte des Schwefels entstehen, die nicht nur unter dem Gesichtspunkt Umwelt, sondern auch im Hinblick auf die Korrosionsbeständigkeit der Anlage verfahrenstechnisch in besonderer Weise berücksichtigt werden müssen.

Weiterhin ist für wasserlösliche Copolymere die Behandlung mit Cystein nicht möglich, da es zur Vernetzung und damit zur Unlöslichkeit des Produktes führt. Darüberhinaus wirken sich die hohen Mengen an Sulfit, zum Teil 2 bis 3 % bezogen auf die Masse des Produktes, sehr negativ auf die anwendungstechnischen Eigenschaften der Produkte aus.

In der DE 35 39 385 wird daher eine Behandlung von Acrylamidpolymerisaten und - copolymerisaten mit Aminen beansprucht.

In der DE 19752127 und DE 19752128 ist die Zugabe von Ammoniak und Aminen bzw. Ammoniumsalzen zur Monomerlösung beschrieben. Der Nachteil dieses Verfahrens liegt darin, daß das Produkt bei mehr als 120 °C getrocknet werden muß, um die gewünschte Wirkung zu erhalten. Dies führt zu einer deutlich verschlechterten Löslichkeit für wasserlösliche Polymerisate durch Vernetzungsreaktionen, die bei erhöhter Temperatur auftreten. Sie tritt insbesondere bei Copolymeren auf, die neben Acrylamid noch ionische Comonomere, wie z.B. Natriumacrylat enthalten.

WO 01/25289 beschreibt die Bestrahlung des Polymergels nach Zerkleinerung. Hierbei wird unter Lichtabschluss in Gegenwart eines UV-Initiators polymerisiert und nach der Zerkleinerung wird dieser durch UV-Licht aktiviert. Die gleiche Verfahrensweise wird in WO 01/55228 beschrieben.

Diese Verfahren sind mit folgenden Nachteilen verbunden:
a) Es muß ein zusätzliches Additiv (Photoinitiator) zur Monomerlösung gegeben werden, das für die eigentliche Polymerisation nicht verwendet wird.
b) Die eigentliche Polymerisation muß unter Lichtausschluß durchgeführt werden, was produktionstechnisch sehr aufwendig ist.
c) Wegen der kurzen Wellenlänge besitzt UV-Licht nur eine geringe Eindringtiefe in das Polymergel nach der Zerkleinerung.

US 2004077744 A1 offenbart ein Polymerisationsverfahren für wasserlösliche oder wasserquellbare Polymere, bei dem zweimal mit UV Licht bestrahlt wird, nämlich einmal zur Auslösung der Polymerisation und einmal als eine extra-Behandlung. Wenngleich die Intensität der UV-Bestrahlung in beiden Schritten unterschiedlich ist, handelt es sich in beiden Fällen um UV-Licht.

JP S63295604 A beschreibt ein Verfahren zur Herstellung eines wasserlöslichen Polymers. Dabei wird auch zweimal mit unterschiedlichen Intensitäten bestrahlt, jedes Mal mit einer "chemischen Fluoreszenzlampe". Der Spektralbereich der verwendeten Lampe lässt sich der JP S63295604 A nicht unmittelbar und eindeutig entnehmen. Es ist aber von einem Wellenbereich von 300 nm bis 500 nm die Rede, was im UV Bereich liegt.

Aufgabe der vorliegenden Erfindung war es, ein Verfahren aufzufinden, das zu Polymerisaten, insbesondere auf Basis von Acryläure bzw. Acrylamid führt, die sehr niedrige bzw. keine Restgehalte an Restmonomeren enthalten, wobei der Anteil an nicht umgesetzten Monomeren bereits während der Polymerisation deutlich absenkt werden bzw. bis zur vollständigen Umsetzung geführt werden soll, so daß mit weniger Restmonomerenfänger gearbeitet werden, bzw. auf diese ganz verzichtet werden kann. Weiterhin soll das Verfahren ohne Additive auskommen, die während der Polymerisation keine Funktion erfüllen bzw. das Verfahren soll auf einem endlosen Band in einer kontinuierlichen Art und Weise durchgeführt werden können.

Überraschenderweise wurde gefunden, daß die Aufgabe durch ein Verfahren zur Herstellung von Polymerisaten gelöst wird, bei dem eine in der Polymerisation befindliche Monomerlösung frühestens nach dem Erreichen der Maximaltemperatur der Polymerisation Tₘₐₓ mit elektromagnetischer Strahlung behandelt und anschließend das erhaltene Gel zerkleinert und getrocknet wird.

Vorzugsweise wird die Monomerlösung bis zu einem Umsatz an den Monomeren von 95 Prozent und erfindungsgemäß besonders bevorzugt bis zu einem Umsatz an den Monomeren von 99 Prozent polymerisiert bis die Behandlung mit elektromagnetischer Strahlung vorgenommen wird.

Die erfindungsgemäß einzusetzende Monomerlösung zur Herstellung der Polymerisate umfaßt anionische, nichtionische, aber auch kationische sowie amphiphile ethylenisch ungesättigte Monomere.

Als anionische Monomere können erfindungsgemäß beispielhaft nachfolgend aufgeführte Monomere verwendet bzw. ausgewählt werden:
a.) olefinisch ungesättigte Carbonsäuren, Carbonsäureanhydride und Carbonsäurenitrile, insbesondere Acrylsäure, Methacrylsäure, Itaconsäure, Crotonsäure, Glutaconsäure, Maleinsäure, Maleinanhydrid, Fumarsäure sowie die wasserlöslichen Ester und/oder Alkali-, Erdalkali- und Ammoniumsalze derselben
b.) olefinisch ungesättigte Sulfonsäuren, insbesondere aliphatische und/oder aromatische Vinylsulfonsäuren, beispielsweise Vinylsulfonsäure, Allylsulfonsäure, Styrolsulfonsäure, Acryl- und Methacrylsulfonsäuren, insbesondere Sulfoethylacrylat, Sulfoethylmethacrylat, Sulfopropylacrylat, Sulfopropylmethacrylat, 2-Hydroxy-3-methacryloxypropylsulfonsäure und 2-Acrylamido-2-methylpropansulfonsäure sowie die wasserlöslichen Alkali-, Erdalkali- und Ammoniumsalze derselben
c.) olefinisch ungesättigte Phosphonsäuren, insbesondere z.B. Vinyl- und Allylphosphonsäure sowie die wasserlöslichen Alkali-, Erdalkali- und Ammoniumsalze derselben
d.) sulfomethylierte und/oder phosphonomethylierte Acrylamide sowie die wasserlöslichen Alkali-, Erdalkali- und Ammoniumsalze derselben.

Vorzugsweise werden olefinisch ungesättigte Carbonsäuren und Carbonsäureanhydride, insbesondere Acrylsäure, Methacrylsäure, Itaconsäure, Crotonsäure, Glutaconsäure, Maleinsäure, Maleinanhydrid, Fumarsäure sowie die wasserlöslichen Ester und/oder Alkali-, Erdalkali- und Ammoniumsalze derselben als anionische Monomere eingesetzt, wobei die wasserlöslichen Alkalisalze der Acrylsäure, insbesondere deren Natrium- und Kaliumsalze sowie deren Ammoniumsalze besonders bevorzugt sind.

Nichtionische Monomere zur Herstellung der Polymerisate A sind beispielsweise Verbindungen der allgemeinen Formel (I) 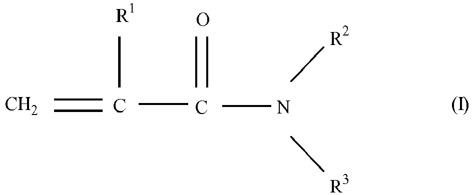 in der
- R¹: für einen Wasserstoff oder einen Methylrest, und
- R² und R³: unabhängig voneinander, für Wasserstoff, für Alkyl- oder Hydroxyalkylrest mit 1 bis 5 C-Atomen, und für eine OH-Gruppe stehen,
eingesetzt werden.

Vorzugsweise werden (Meth)acrylamid, N-Methyl(meth)acrylamid, N-Isopropyl(meth)acrylamid oder N,N-substituierte (Meth)acrylamide, wie N,N-Dimethyl(meth)acrylamid, N,N-Diethyl(meth)acrylamid, N-Methyl-N-ethyl(meth)acrylamid oder N-Hydroxyethyl(meth)acrylamid, eingesetzt.

Erfindungsgemäß vorteilhaft können als nichtionische Monomere, insbesondere die wasserlöslichen bzw. wasserdispergierbaren Derivate der Acryl- und Methacrylsäure wie z.B. Acrylamid, Methacrylamid sowie n-alkylsubstituierte Acrylamide eingesetzt werden, wobei erfindungsgemäß Acrylamid als nichtionisches Monomer ganz besonders bevorzugt ist.

Neben den zuvor aufgeführten nichtionischen Monomeren können auch weitere Monomere erfindungsgemäß verwendet werden, wie z.B. Acrylnitril, Vinylpyridin, Vinylacetat, hydroxygruppenhaltige Ester polymerisationsfähiger Säuren sowie die Hydroxyalkyl-, insbesondere die Hydroxyethyl- und propylester der Acrylsäure und Methacrylsäure.

Als ebenfalls einsetzbare kationische Monomere zur Herstellung der erfindungsgemäßen Polymerisate eignen sich Verbindungen der allgemeinen Formel (II) 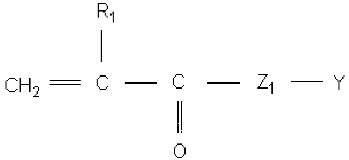 in der
- R₁: für Wasserstoff oder einen Methylrest,
- Z₁: für O, NH oder NR₄ mit R₄ für einen Alkylrest mit 1 bis 4 C-Atomen,
- Y: für eine der Gruppen 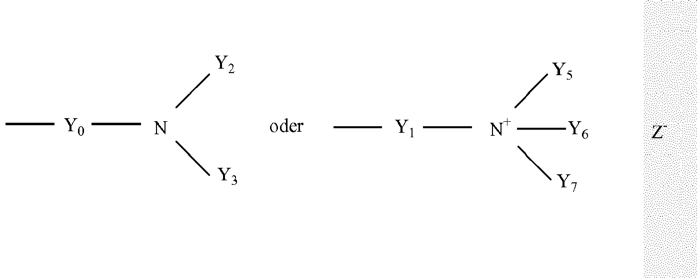
stehen, wobei
Y₀ und Y₁ für einen Alkylenrest oder Hydroxyalkylenrest mit 2 bis 6 C-Atomen,
Y₂, Y₃, Y₄, Y₅, Y₆, Y₇, unabhängig voneinander, für einen Alkylrest mit 1 bis 6 C-Atomen,
Z⁻ für Halogen, Acetat, SO₄CH₃⁻ stehen.

Vorzugsweise sind als protonierte oder quaternierte Dialkylaminoalkyl(meth)acrylate oder-(meth)acrylamide mit C₁ bis C₃ in den Alkyl- bzw. Alkylengruppen, besonders bevorzugt das mit Methylchlorid quaternierte Ammoniumsalz von Dimethylaminoethyl(meth)acrylat, Dimethylaminopropyl(meth)acrylat, Dimethylaminoethyl(meth)acrylat, Diethylaminoethyl(meth)acrylat, Dimethylaminomethyl(meth)acrylat Dimethylaminoethyl(meth)acrylamid und/ oder Dimethylaminopropyl-(meth)acrylamid geeignet.

Als amphiphile Monomere eignen sich gegebenenfalls Verbindungen der allgemeinen Formel (III) oder (IV) 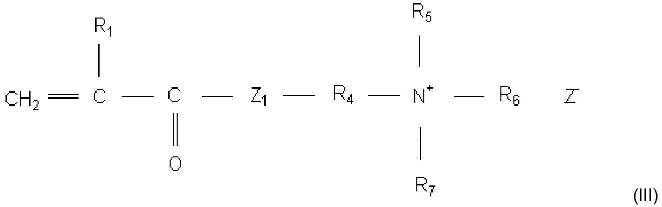 wobei
- Z₁: für O, NH, NR₄ mit R₄ für Alkyl mit 1 bis 4 Kohlenstoffatomen,
- R₁: für Wasserstoff oder einen Methylrest,
- R₄: für Alkylen mit 1 bis 6 Kohlenstoffatomen,
- R₅ und R₆: unabhängig voneinander, für einen Alkylrest mit 1 bis 6 Kohlenstoffatomen,
- R₇: für einen Alkyl-, Aryl- und/oder Aralkyl-Rest mit 8 bis 32 Kohlenstoffatomen und
- Z⁻: für Halogen, Pseudohalogen, SO₄CH₃⁻ oder Acetat stehen oder 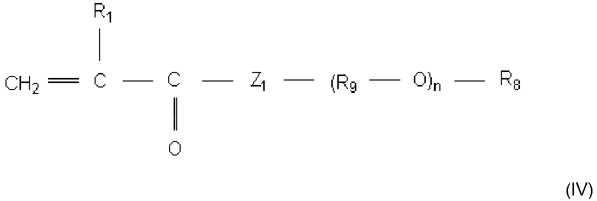
wobei
- Z₁: für O, NH, NR₄ mit R₄ für Alkyl mit 1 bis 4 Kohlenstoffatomen,
- R₁: für Wasserstoff oder einen Methylrest,
- R₈: für Wasserstoff, einen Alkyl-, Aryl- und/oder Aralkyl-Rest mit 8 bis 32 Kohlenstoffatomen,
- R₉: für einen Alkylenrest mit 2 bis 6 Kohlenstoffatomen
- n: für eine ganze Zahl von 1 und 50
stehen, eingesetzt werden.

Vorzugsweise sind dies Umsetzungsprodukte von (Meth)acrylsäure mit Polyethylenglykolen (10 bis 40 Ethylenoxideinheiten), die mit Fettalkohol verethert sind, bzw. die entsprechenden Umsetzungsprodukte mit (Meth)acrylamid.

Zur Herstellung der nach dem erfindungsgemäßen Verfahren hergestellten Polymerisate wird für die polymerisierende Monomerlösung im Falle wasserlöslicher Polymerisate eine Monomerzusammensetzung gewählt, die aus 0 bis 100 Gew.-%, vorzugsweise aus 5 bis 70 Gew.-%, besonders bevorzugt 15 bis 40 Gew.-%, jeweils bezogen auf die Gesamtmenge an Monomeren, vorzugsweise anionischer Monomeren besteht. Ganz besonders bevorzugt wird zur Herstellung der wasserlöslichen Polymerisate eine Mischung aus nichtionischen Monomeren, vorzugsweise Acrylamid und anionischen Monomeren, insbesondere olefinisch ungesättigte Carbonsäuren und Carbonsäureanhydride, vorzugsweise Acrylsäure, Methacrylsäure, Itaconsäure, Crotonsäure, Glutaconsäure, Maleinsäure, Maleinanhydrid, Fumarsäure sowie die wasserlöslichen Ester und/oder Alkali-, Erdalkali- und Ammoniumsalze derselben eingesetzt, wobei Acrylsäure als anionisches Monomer besonders bevorzugt ist.
Bevorzugt ist auch eine Mischung von Acrylsäure mit Alkyl(meth)acrylaten und/oder Alkyl(meth)acrylamiden.

Wasserquellbare Polymerisate als wasserabsorbierende Absorptionsmittel werden meistens unter Verwendung von mindestens einem Vernetzer erhalten. Als Vernetzer werden solche Verbindungen verwendet, die mindestens zwei oder mehr funktionelle Gruppen (Doppelbindungen, Epoxygruppen) enthalten und in der Lage sind, sich während der Polymerisation in die wachsenden Polymerketten einzubauen. Damit entstehen im Polymerisat an verschiedenen Stellen Vernetzungspunkte, die die einzelnen Polymerketten zusammenhalten und bewirken, daß in einer Flüssigkeit die Polymerpartikel nur quellen können und sich nicht in der Flüssigkeit auflösen. Durch die chemische Struktur des Vernetzers, die Anzahl der Vernetzungsstellen, aber auch durch ihre Verteilung in den Polymerketten werden die Eigenschaften des vernetzten Polymeren bestimmt. Bei optimalem Einbau des Vernetzers in das Polymerisat entstehen vernetzte Polymerisate mit gleichmäßig verteilten Vernetzungsstellen, so daß kaum nichtvernetzte Regionen oder sogar unvernetzte (d.h. wasserlösliche) niedermolekulare Anteile im Polymerisat vorhanden sind. Eine gleichmäßige Verteilung der Vernetzungsstellen im Polymerisat führt zu einem Produkt, das auch ein optimales Rückhaltevermögen für wäßrige Flüssigkeiten sowie eine optimale Gelfestigkeit im gequollenen Zustand aufweist.

Bei den erfindungsgemäß einzusetzenden Monomeren für die Monomerlösung zur Herstellung von wasserquellbaren Polymerisaten handelt es sich in erster Linie um Acrylsäure, Methacrylsäure, Acrylamid sowie Methacrylamid, die alleine zu Homopolymerisaten oder zu Mischpolymerisaten polymerisiert werden, ferner aber auch andere wasserlösliche Monomeren, wie Acrylnitril, Methacrylnitril, N,N-Dimethylacrylamid, Vinylpyridin, Vinylacetat sowie weitere wasserlösliche polymerisationsfähige Säuren und ihre Salze, insbesondere die Malein-, Fumar-, Itacon-, Vinylsulfon- oder Acrylamidomethylpropansulfonsäure; ferner hydroxygruppenhaltige Ester polymerisationsfähiger Säuren, insbesondere die Hydroxyalkylester, vorzugsweise Hydroxyethyl- und Hydroxypropylester der Acryl- und der Methacrylsäure können verwendet werden; weiter aminogruppenhaltige und ammoniumgruppenhaltige Ester und Amide polymerisationsfähiger Säuren wie die Dialkylaminoester, insbesondere die Dimethyl- und die Diethylaminoalkylester der Acryl- und der Methacrylsäure, sowie die Trimethyl- und Trimethylammoniumalkylester sowie die entsprechenden Amide. Die vorstehenden Monomeren können allein zu Homopolymerisaten oder untereinander gemischt zu Mischpolymerisaten polymerisiert werden.

In geringen Mengen können noch zusätzlich wasserunlösliche Monomere mit den vorstehenden Monomeren copolymerisiert werden, wie z.B die Ester der Acryl- und/oder Methacrylsäure mit C₁-C₁₀-Alkoholen, Styrol und alkylierte Styrole. Im allgemeinen liegt der Anteil an den wasserlöslichen Monomeren in der Monomerlösung bei 60 bis 100 Gew%, insbesondere bei 80 bis 100 Gew.-%, bezogen auf die Gesamtheit der Monomeren, wobei Acrylsäure als Monomer bevorzugt ist. Die wasserunlöslichen (hydrophoben) Monomere machen in der Regel 0 bis 40 Gew% der Monomeren aus.

Die für die Polymerisation benötigte Acrylsäure bzw. deren Derivate werden destillativ gereinigt und im Anschluß hieran vorzugsweise zu wenigstens 30 Mol-% neutralisiert. Sobald sich das gewünschte Neutralisationsverhältnis eingestellt hat, ist die Polymerisationsreaktion unmittelbar zu starten. Die einzusetzenden Monomeren, insbesondere Acrylsäure sollten vor der Polymerisation immer frisch gereinigt sein, da z.B. eine lange Lagerung der Acrylsäuremonomeren verbunden mit einer hohen Lagerungstemperatur zu einem unerwünschten Anstieg des Diacrylsäuregehaltes in der Monomerlösung führt, der dann einen gesteigerten Restmonomerengehalt im Polymerisat bewirken kann.

Die Neutralisation der Säuregruppen in den wäßrigen Monomerlösungen erfolgt mit Basen nach bekannten Methoden, wobei der Neutralisationsgrad vorzugsweise bei wenigstens 30 Mol-%, insbesondere zwischen 50 und 100 Mol-% und besonders bevorzugt zwischen 70 und 95 Mol-% liegt. Als Basen kommen für die Neutralisation alle gängigen anorganischen und organischen Verbindungen in Betracht, die sowohl allein als auch in Mischungskombinationen zur Neutralisation eingesetzt werden können. Bevorzugte Neutralisationsmittel sind neben Natrium- und Kalilauge sowie den entsprechenden Carbonaten auch basische Stickstoffverbindungen, wie beispielsweise Ammoniak bzw. Ammoniumhydroxid, Ammoniumsalze sowie Aminoverbindungen wie aliphatische, cycloaliphatische, heterocyclische und aromatische Mono- und Polyamine, Hydroxylamine und Alkanolamine. Besonders bevorzugte Stickstoffverbindungen sind Ammoniak bzw. Ammoniumhydroxid, Hydroxylamin, Ethanolamin, Diethanolamin sowie deren Gemische.

Zur Herstellung wasserquellbarer Polymerisate können zusammen mit den o.g. Monomeren in geringen Anteilen vernetzende Monomeren wie z.B. Monomere mit mehr als einer reaktionsfähiger Gruppe im Molekül polymerisiert werden. Solche Vernetzer, die mindestens zwei oder mehr funktionelle Gruppen, z.B. Doppelbindungen, Epoxygruppen etc. enthalten, sind in der Lage, sich während der Polymerisation in die wachsenden Polymerketten einzubauen. Damit entstehen im Polymerisat an verschiedenen Stellen Vernetzungspunkte, die die einzelnen Polymerketten zusammenhalten und bewirken, daß in einer Flüssigkeit die Polymerpartikel nur quellen können und sich nicht in der Flüssigkeit auflösen. Durch die chemische Struktur des Vernetzers, die Anzahl der Vernetzungsstellen, aber auch durch ihre Verteilung in den Polymerketten werden die Eigenschaften des vernetzten Polymeren bestimmt. Bei optimalem Einbau des Vernetzers in das Polymerisat entstehen vernetzte Polymerisate mit gleichmäßig verteilten Vernetzungsstellen, so daß kaum nichtvernetzte Regionen oder sogar unvernetzte (d.h. wasserlösliche) niedermolekulare Anteile im Polymerisat vorhanden sind. Eine gleichmäßige Verteilung der Vernetzungsstellen im Polymerisat führt zu einem Produkt, das auch ein optimales Rückhaltevermögen für wäßrige Flüssigkeiten sowie eine optimale Gelfestigkeit im gequollenen Zustand aufweist.

Als vernetzende Monomere seien hier exemplarisch mehrfunktionelle Monomere, z.B. Amide wie das Methylenbisacryl- bzw. -methacrylamid oder Ethylenbisacrylamid, ferner Ester von Polyolen, wie Diacrylate oder Triacrylate, z.B. Butandiol- oder Ethylenglykoldiacrylat, Polyglykol-di-(meth)acrylate, Trimethylolpropantriacrylat, Di- und Triacrylatesterdes, vorzugsweise mit 1 bis 30 Mol oxalkylierten (ethoxylierten) Trimethylolpropans, Acrylat- und Methacrylatester von Glycerin und Pentaerythrit, sowie des vorzugsweise mit 1 bis 30 Mol oxethylierten Glycerins und Pentaerythrits, ferner Allylverbindungen wie Allyl(meth)acrylat, Triallylcyanurat, Maleinsäurediallylester, Polyallylester, Tetraallyloxiethan, Triallylamin, Tetraallylethylendiamin, Allylester der Phosphorsäure bzw. phosphorigen Säure, ferner vernetzungsfähige Monomere, wie die N-Methylolverbindungen von Amiden wie dem Methacrylamid bzw. Acrylamid und die davon abgeleiteten Ether genannt. Der Anteil an den vernetzenden Comonomeren liegt bei 0 bis 10 Gew%, bevorzugt bei 0,01 bis 3,0 Gew%, bezogen auf die Gesamtheit der Monomeren.

Die Polymerisation der Monomerlösung erfolgt mit üblichen, eine radikalische Polymerisation auslösenden Initiatoren bzw. Redoxsystemen. Um eine wirtschaftliche Herstellung der Polymerisate zu erreichen, wird die Polymerisation vorzugsweise mit solchen Initiatoren durchgeführt, die bei relativ niedrigen Temperaturen zerfallen.
Die Polymerisation wird üblicherweise als adiabatische Polymerisation durchgeführt, wobei die Reaktion vorzugsweise sowohl mit einem Redoxsystem als auch mit einem Photoinitiator gestartet werden kann. Auch ist eine Kombination von beiden Start-Varianten möglich.

Das Redox-Initiatorsystem besteht vorzugsweise aus mindestens zwei Komponenten, nämlich einem organischen oder anorganischen Oxidationsmittel und einem organischen oder anorganischen Reduktionsmittel. Hierbei werden häufig dabei Verbindungen mit Peroxideinheiten verwendet, beispielsweise anorganische Peroxide, insbesondere Alkalimetall- und Ammoniumpersulfat, Alkalimetall- und Ammoniumperphosphate, Wasserstoffperoxid und dessen Salze, z.B. Natriumperoxid und Bariumperoxid oder organische Peroxide wie z.B. Benzoylperoxyd, Butylhydroperoxid oder Persäuren wie Peressigsäure. Daneben können aber auch andere Oxidationsmittel eingesetzt werden, z.B. Kaliumpermanganat, Natrium- und Kaliumchlorat, Kaliumdichromat usw. Als Reduktionsmittel können schwefelhaltige Verbindungen wie Sulfite, Thiosulfate, Sulfinsäure, organische Thiole, insbesondere Ethylmercaptan, 2-Hydroxyethanthiol, 2-Mercaptoethylammoniumchlorid, Thioglykolsäure und andere verwendet werden. Weiterhin ist der Einsatz von Ascorbinsäure und niedervalenten Metallsalzen möglich, wie z.B. [Kupfer(I); Mangan(II); Eisen(II)]. Auch Phosphorverbindungen können verwendet werden. Exemplarisch sei hierfür Natriumhypophosphit genannt.

Im Falle einer Photopolymerisation wird die Reaktion mit UV-Licht gestartet, das den Zerfall des Starters bewirkt. Als Starter können Benzoin- und Benzoinderivate, wie Benzoinether, Benzil und seine Derivate, wie Benzilketale, Acryldiazoniumsalze, Azoinitiatoren wie z.B. 2,2'-Azobis(isobutyronitril), 2,2'-Azobis(2-amidinopropan)-hydrochlorid oder Acetophenonderivate verwendet werden. Die Menge der oxidierenden und der reduzierenden Komponente kann sich im Bereich zwischen 0,00005 und 0,5 Gewichtsprozent, vorzugsweise von 0,001 bis 0,1 Gewichtsprozent bezogen auf die Monomerlösung und für Photoinitiatoren zwischen 0,001 und 0,1 Gewichtsprozent, bevorzugt 0,002 bis 0,05 Gewichtsprozent bewegen.

Die Polymerisation wird bevorzugt in wässriger Lösung diskontinuierlich in einem Polymerisationsgefäß oder kontinuierlich auf einem endlosen Band, z.B. gemäß DE 35 44 770 durchgeführt, auf die vollinhaltlich Bezug genommen und hiermit in die Beschreibung dieser Patentanmeldung mitaufgenommen wird.

Bei einem praktisch adiabatischen Verlauf der Polymerisation entsteht bei entsprechender Anfangskonzentration von 15 bis 50 Gewichtsprozent ein wässriges Polymergel. Durch die Wahl der Anfangsmonomerkonzentration und der entsprechenden Starttemperatur vorzugsweise im Bereich von -20 bis +50°C, besonders bevorzugt bei -10 bis +10°C, kann das Temperaturprofil der Reaktion so gesteuert werden, daß sie gut beherrschbar ist.

Erfindungswesentlich für das erfindungsgemäße Verfahren ist, das die in Polymerisation befindliche Monomerlösung mit elektromagnetischer Strahlung, vorzugsweise mit Infrarot-Strahlung frühestens nach dem Erreichen der Maximaltemperatur der Polymerisation behandelt wird, da zu diesem Zeitpunkt von einem sehr hohen Umsatz der Monomere ausgegangen werden kann. Vorzugsweise wird das bei der Polymerisation erhaltene Polymergel bei einem Monomerenumsatz von 95% und ganz besonders bevorzugt von mindestens 99% mit elektromagnetischer Strahlung behandelt.

Als elektromagnetische Strahlung kann beispielsweise IR-Strahlung, Mikrowellenstrahlung, VIS- aber auch UV-Strahlung für das erfindungsgemäße Verfahren verwendet werden, insbesondere im Frequenzbereich v = 10⁹ bis 10¹⁶ Hz entsprechend einem Wellenlängenbereich von λ = 35 cm bis 3 nm, wobei der Einsatz von IR-Strahlung besonders bevorzugt ist. Bei Verwendung von IR-Strahlung ist der Wellenlängenbereich von λ = 750 bis 25000 nm bevorzugt bzw. für den Einsatz von sichtbarem Licht (VIS-Strahlung) liegt der bevorzugte Wellenlängenbereich bei λ = 380 bis 750 nm. Bei Verwendung von UV-Strahlung wird diese bevorzugt im Wellenlängenbereich λ = 172 bis 380 nm entsprechend einem Frequenzbereich von v = 900 MHZ bis 140 GHz eingesetzt.

Im Hinblick auf den möglichen Einsatz von UV-Strahlung im erfindungsgemäßen Verfahren ist zu betonen, daß es bekannt ist, im Falle von Photopolymerisationen, bei denen die Reaktion mit UV-Licht, d.h. UV-Strahlung gestartet wird, die Bestrahlung mit diesem UV-Licht während der gesamten Polymerisationsreaktion aufrechtzuerhalten, um den vollständigen Zerfall des Photoinitiators zu gewährleisten. Völlig überraschend wurde jedoch auch hier gefunden, daß durch eine in diesem Falle zusätzliche erfindungsgemäße Behandlung frühestens nach dem Erreichen der Maximaltemperatur der Polymerisation mit elektromagnetischer Strahlung, wobei diese elektromagnetische Strahlung eine größere Wellenlänge aufweist als das zum Zerfall des Photoinitiators verwendete UV-Licht, Polymerisate, insbesondere auf Basis von Acryläure bzw. Acrylamid erhalten werden, die sehr niedrige bzw. keine Restgehalte an Restmonomeren enthalten. Für diese erfindungsgemäße Ausführungsform ist wesentlich, daß die verwendete elektromagnetische Strahlung eine größere Wellenlänge aufweist als das zum Zerfall des Photoinitiators verwendete UV-Licht. Erfindungsgemäß wird hierfür die IR Strahlung verwendet.

Die Dauer der Behandlung mit elektromagnetischer Strahlung im erfindungsgemäßen Verfahren beträgt abhängig von der Art der verwendeten Strahlung 0 bis 60 Minuten, bevorzugt 3 bis 25 Minuten und besonders bevorzugt 5 bis 20 Minuten.

Nach dem Ende der Polymerisation erfolgt eine Zerkleinerung des als Gel vorliegenden Polymerisates. Das zerkleinerte Gel wird nun diskontinuierlich beispielsweise in einem Umlufttrockenschrank bei 70 bis 150 °C, bevorzugt bei 80 bis 130 °C getrocknet. Kontinuierlich kann die Trocknung in den gleichen Temperaturbereichen auf einem Bandtrockner oder in einem Wirbelbettrockner geschehen.

Die erfindungsgemäß hergestellten Polymerisate werden nach der Trocknung auf die gewünschte Kornfraktion gemahlen und anschließend in die für die verschiedenen Anwendungen erforderlichen Siebfraktionen aufgeteilt. Beispielsweise sind für die Anwendung von vernetzten Polymerisaten als Absorptionsmittel im Hygienebereich Kornverteilungen von 0,2 bis 1 mm bevorzugt. Zur Verwendung im Agrarbereich und der Hortikultur werden Produkte mit einer Kornverteilung von 0,2 bis 3 mm eingesetzt, wohingegen für lösliche Polymere bei Verwendung als Flockungsmittel Produkte im Bereich von 0,1 bis 1,2 mm bevorzugt sind. Insbesondere um ein schnelles Auflösen des Produktes zu erreichen, sollten mindestens 90 Prozent des Produktes unter 2,0 mm, bevorzugt 90 Prozent unter 1,5 mm groß sein.

In einer bevorzugten Ausgestaltung der Erfindung, insbesondere zur Herstellung wasserquellbarer superabsorbierender Polymerisate mit dem gewünschten Eigenschaftsprofil von hoher Retentionskapazität, hoher Gelstärke und hohem Aufnahmevermögen unter Druck, werden die Oberflächen dieser absorbierenden Polymerisate nachträglich behandelt, damit den hohen Anforderungen der Personal Care Industrie an solche Polymerisate bei ihrer Verwendung in Hygieneprodukten entsprochen werden kann. Zu einer solchen Nachvernetzung, die zu einer deutlichen Verbesserung der Gelstabilität, der Flüssigkeitsaufnahme unter Druck und der Aufnahmegeschwindigkeit, verbunden mit einem niedrigen Restmonomerengehalt, führt, werden vornehmlich Verbindungen eingesetzt, die mindestens zwei funktionelle Gruppen besitzen und die die funktionellen Gruppen des Polymerisates an der Oberfläche der Polymerteilchen vernetzen können.

Hier sind Vernetzer mit Alkohol-, Amin-, Aldehyd-, Glycidyl- und Epichlorfunktionen wie mehrwertige Alkohole, Polyepoxyverbindungen, Polyglycidylverbindungen, Polyamine, polyfunktionelle Aziridinverbindungen, Polyaldehyde, Polyisocyanate Polyoxaziline, Alkylencarbonate und mehrwertige Metallverbindungen zu nennen, wobei auch Vernetzermoleküle mit mehreren verschiedenen Funktionen verwendet werden können. Beispielhaft seien die folgenden Nachvernetzungsmittel genannt: Ethylenglykol, Diethylenglykol, Triethylenglykol, Polyethylenglykol, Glycerin, Polyglycerin, Propylenglykol, Diethanolamin, Triethanolamin, Propylenoxid, Blockcoploymere aus Ethylen- und Propylenoxid, Sorbitanfettsäureester, ethoxilierte Sorbitanfettsäureester, Trimethylolpropan, ethoxiliertesTrimethylolpropan, Pentaerythrit, ethoxiliertes Pentaerythrit, Polyvinylalkohol, Sorbit, Ethylencarbonat, Propylencarbonat und Polyepoxide wie z.B. Ethylenglykoldiglycidylether, wobei Ethylencarbonat besonders bevorzugt ist.

Die genannten Vernetzer können allein, aber auch als Mischungen mehrer der vorgenannten Vernetzer, sowohl direkt als auch als wäßrige alkoholische oder wäßrige Lösung, zur Oberflächenvernetzung der Polymerisate verwendet werden. Das Nachvernetzungsmittel wird dabei üblicherweise in einer Menge von 0,01 bis 10 Gew.-%, bevorzugt 0,1 bis 5 Gew.-% und besonders bevorzugt 0,1 bis 1,0 Gew.-%, bezogen auf das nachzuvernetzende Polymerisat, eingesetzt.

Um die gewünschten Eigenschaften zu erhalten, ist eine gleichmäßige Verteilung des Nachvernetzungsmittels auf den Polymerteilchen erforderlich. Geeignete Mischaggregate zum Aufbringen des Nachvernetzungsmittels sind z.B. Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende Mischer, in denen die Polymerisatteilchen als Pulver mittels rotierender Messer in schneller Frequenz gemischt werden (Schugi-Mischer). Nachdem das Nachvernetzungsmittel, vorzugsweise in Form einer Lösung mit den Polymerteilchen vermischt worden ist, wird zur Oberflächenvernetzung auf Temperaturen von 80 bis 250 °C, bevorzugt auf 135 bis 250 °C und besonders bevorzugt auf 150 bis 200 °C erhitzt. Die optimale Zeitdauer ist vom Typ des verwendeten Nachvernetzers abhängig und kann für jeden Vernetzer in wenigen Versuchen leicht ermittelt werden. Die Nachvernetzungsdauer wird durch den Zeitpunkt limitiert, an dem das gewünschte Eigenschaftsprofil des superabsorbierenden Polymersates infolge der auftretenden Hitzeschädigung wieder zerstört wird. Üblicherweise beträgt die Nachvernetzungsdauer bei Temperaturen von 180 °C weniger als 30 Minuten.

Besonders vorteilhaft ist, daß das erfindungsgemäße Verfahren zur Herstellung von Polymergelen verwendet werden kann, deren Herstellung üblicherweise nach dem Bandverfahren erfolgt, weil das erfindungsgemäße Verfahren auf den bestehenden Bandpolymerisationsanlagen lediglich durch die Nachrüstung der entsprechenden Strahlungsvorrichtungen, beispielsweise von Infrarot-Strahlungseinrichtungen ohne weitere Modifikation der bestehenden Anlagentechnik durchgeführt werden kann. Hierbei führt das erfindungsgemäße Verfahren zu deutlich niedrigeren Restgehalten an Restmonomeren, insbesondere an Acrylamid im Vergleich zur bekannten Verfahrenweise ohne Bestrahlung.

Vorteilhaft lassen sich die nach dem erfindungsgemäßen Verfahren hergestellten Polymerisate vielfältig einsetzen. Beispielsweise können wasserquellbare Polymerisate in der Personal Care Industrie bzw. Hygieneindustrie bei der Herstellung von Hygieneartikeln, wie z.B. absorbierenden Sanitärartikeln für die Kinder- und Erwachsenhygiene in Form von Babywindeln, Damenbinden, Tampons, Inkontinenzartikeln sowie Produkten zur Wundabdeckung, in die sie eingearbeitet sind, verwendet werden.

Weiterhin können solche wasserquellbaren Polymerisate in der Verpackungsindustrie verwendet werden, beispielsweise in Nonwoven-Produkten, in denen diese wasserquellbaren Polymerisate eingearbeitet sind, z.B. als Verpackungskomponente, insbesondere in Form von Saugeinlagen für Fisch- und Fleischschalen verwendet werden.

Darüberhinaus werden die wasserquellbaren Polymerisate in Form von Granulaten in der Agrartechnik bzw. Land- und Gartenwirtschaft verwendet, insbesondere als Bodenhilfsstoffe zur Wasser- und Nährstoffspeicherung, künstliche Böden zur Pflanzenzüchtung sowie als Wurzelschutzgele. In der Kabelindustrie bzw. Nachrichtentechnik werden solche wasserquellbaren Polymerisate als flüssigkeitsabsorbierende Komponenten bzw. als Additive in strom- oder lichtleitenden Kabeln eingesetzt.

In wasserlöslicher Form können die Polymerisate beispielsweise als Flockungshilfsmit tel, vorzugsweise zur Klärung kommunaler und industrieller Abwässer, als Bohrspülmittel in der Erdölförderung, als Bodenverbesserungsmittel, Nahrungsmittelzusätze, Verdickungsmittel, Dispergierhilfsmittel und Retentionshilfsmittel verwendet werden.

Im folgenden wird die Erfindung anhand von Beispielen erläutert.

### Beispiele

Folgende Abkürzungen werden nachfolgend verwendet:
- ABAH:: 2,2'-Azobis(2-amidinopropan)-hydrochlorid
- DIMAPA-Quat:: 3-Dimethylammoniumpropyl(meth)acrylamid, das mit Methylchlorid quaterniert wurde
- ADAME-Quat:: 2-Dimethylammoniumethyl(meth)acrylat, das mit Methylchlorid quaterniert wurde
- Versenex 80: Handelsprodukt der Fa. The DOW Chemical Company

### I. Herstellung von Copolymeren aus Natriumacrylat und Acrylamid mit einem Massenanteil von 40 Prozent Natriumacrylat

### Vergleichsbeispiel:

In einem Polymerisationsgefäß wurden zunächst 360,0 g 50 Gew.-% wässrige Acrylamidlösung vorgelegt und mit 445,9 g Wasser sowie 250 mg Versenex 80 vermischt. Nach der Zugabe von 91,9 g Acrylsäure wurde mit 102,1 50 Gew.-% wässriger NaOH-Lösung auf pH 6,0 neutralisiert, auf -5°C abkühlt und mit Stickstoff ausgeblasen. Nach der Zugabe von 0,45 g ABAH wurde die Polymerisation mit UV-Licht gestartet. Binnen 25 Minuten steigt die Reaktionstemperatur von -5°C auf Tₘₐₓ = 80 °C. Anschließend ließ man 25 Minuten nachreagieren. Das erhaltene Polymerisat wurde mit einem Fleischwolf zerkleinert und bei 100°C für 90 Minuten getrocknet. Das Produkt wurde dann auf eine Kornfraktion von 90 bis 1400 µm vermahlen.

Das Produkt besitzt eine Viskosität von 210 mPas, gemessen mit einem Brookfield-Viskosimeter an einer 0,5 %-igen Lösung in 10 %-iger NaCI-Lösung, wobei die Lösezeit eine Stunde betrug.

### Beispiele 1 bis 3:

Die Polymerisation erfolgte wie im Vergleichsbeispiel, nur wurde während der Nachreaktionszeit von 20 bis 25 Minuten, d.h. nach dem Erreichen von Tₘₐₓ mit einem kurzwelligem IR-Strahler belichtet, wobei die mittleren Wellenlängen 1 bis 1,5 mm betrugen. Die Regelung erfolgte über die Oberflächentemperatur. Die Aufarbeitung erfolgte analog zum Vergleichsbeispiel. Die Viskosität lag ebenfalls bei 210 mPas, gemessen mit einem Brookfield-Viskosimeter an einer 0,5 %-igen Lösung in 10 %-iger NaCl-Lösung bestimmt, wobei die Lösezeit eine Stunde betrug.

In der folgenden Abbildung sind die ermittelten Restmonomerengehalte aus den Endprodukten gemäß Vergleichsbeispiel bzw. der Beispiele 1 bis 3 dargestellt:

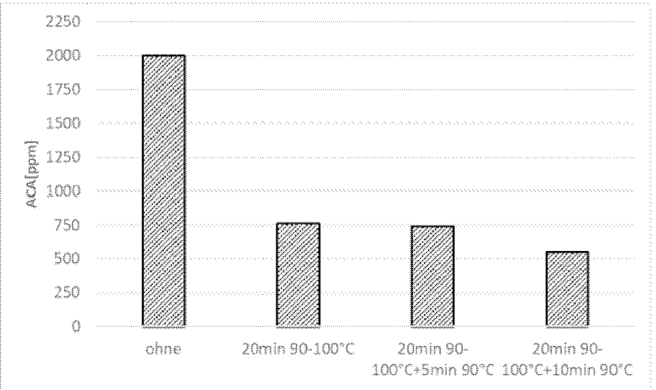

### II. Herstellung kationischer Copolymeren aus ADAME-Quat und Acrylamid mit 35 Massenprozent ADAME-Quat

### Beispiele 4 bis 5:

In einem Polymerisationsgefäß wurden zunächst 1040,0 g 50 proz. wässrige Acrylamidlösung vorgelegt und mit 1109,0 g Wasser sowie 0,75 g Versenex 80 vermischt. Nach der Zugabe von 350,0 g 80 %-igeproz. ADAME-Quat- Lösung wurde mit 0,25 g 50 %-iger Schwefelsäure auf pH 5,0 eingestellt, auf-0°C abkühlt und mit Stickstoff ausgeblasen. Nach der Zugabe von 1,25 g ABAH (2,2'-Azobis(2-methylpropionamidin)-dihydrochlorid) wurde die Polymerisation mit UV-Licht gestartet. Binnen 25 Minuten steigt die Temperatur von -5°C auf 80 °C. Bei Tₘₐₓ. wird das Gel mit einem IR-Strahler für 10 min. bestrahlt (mittlere Wellenlänge 1 bis 1,5 mm). Das erhaltene Polymer wurde mit einem Fleischwolf zerkleinert und bei 100°C für 90 Minuten getrocknet. Das Produkt wurde auf eine Kornfraktion von 90-1400 µm vermahlen. Die Viskosität betrug 705 mPas, gemessen als 1,0 %-ige Lösung in 10 %-iger NaCl-Lösung.

Ein entsprechender Vergleichsansatz wurde ohne Bestrahlung durchgeführt.
Das erhaltene Polymer wurde ebenfalls mit einem Fleischwolf zerkleinert, bei 100°C für 90 Minuten getrocknet und das Produkt auf eine Kornfraktion von 90-1400 µm vermahlen. Die Viskosität betrug 680 mPas, gemessen als 1,0 %-ige Lösung in 10 %-iger NaCl-Lösung.

Im erfindungsgemäßen Beispiel 4 lagen die Restmonomere bei 640 ppm, im Vergleichsbeispiel 5 bei 1500 ppm.

## Patentansprüche

1. Verfahren zur Herstellung von Polymerisaten durch Photopolymerisation, bei dem eine Reaktion mit UV-Licht gestartet wird und bei dem Bestrahlung mit diesem UV-Licht während der gesamten Polymerisationsreaktion aufrechterhalten wird, um den vollständigen Zerfall eines Photoinitiators zu gewährleisten, bei dem eine in Polymerisation befindliche Monomerlösung frühestens nach dem Erreichen der Maximaltemperatur der Polymerisation mit elektromagnetischer Strahlung behandelt, anschließend das erhaltene Gel zerkleinert und getrocknet wird,
**dadurch gekennzeichnet,**
**dass** die elektromagnetische Strahlung eine größere Wellenlänge aufweist als das zum Zerfall des Photoinitiators verwendete UV-Licht,
und **dass** es sich bei der elektromagnetischen Strahlung um IR-Strahlung handelt.

2. Verfahren zur Herstellung von Polymerisaten nach Anspruch 1, bei dem die Monomerlösung bis zu einem Umsatz an den Monomeren von 95 Prozent polymerisiert wird.

3. Verfahren zur Herstellung von Polymerisaten nach Anspruch 1, bei dem die Monomerlösung bis zu einem Umsatz an den Monomeren von 99 Prozent polymerisiert wird.

4. Verfahren zur Herstellung von Polymerisaten nach einem der Ansprüche 1 bis 3, **dadurch gekennzeichnet, daß** die Monomerlösung anionische, nichtionische, kationische und/oder amphiphile ethylenisch ungesättigte kationische Monomere umfaßt.

5. Verfahren zur Herstellung von Polymerisaten nach Anspruch 4, **dadurch gekennzeichnet, daß** die Monomerlösung mindestens ein anionisches Monomer umfaßt, daß aus den Gruppen
a.) olefinisch ungesättigte Carbonsäuren und Carbonsäureanhydride, insbesondere Acrylsäure, Methacrylsäure, Itaconsäure, Crotonsäure, Glutaconsäure, Maleinsäure, Maleinanhydrid, Fumarsäure sowie die wasserlöslichen Alkali-, Erdalkali- und Ammoniumsalze derselben
b.) olefinisch ungesättigte Sulfonsäuren, insbesondere aliphatische und/oder aromatische Vinylsulfonsäuren, beispielsweise Vinylsulfonsäure, Allylsulfonsäure, Styrolsulfonsäure, Acryl- und Methacrylsulfonsäuren, insbesondere Sulfoethylacrylat, Sulfoethylmethacrylat, Sulfopropylacrylat, Sulfopropylmethacrylat, 2- Hydroxy-3-methacryloxypropylsulfonsäure und 2-Acrylamido-2-methylpropansulfonsäure sowie die wasserlöslichen Alkali-, Erdalkali- und Ammoniumsalze derselben
c.) olefinisch ungesättigte Phosphonsäuren, insbesondere z.B. Vinyl- und Allylphosphonsäure sowie die wasserlöslichen Alkali-, Erdalkali- und Ammoniumsalze derselben
d.) sulfomethylierte und/oder phosphonomethylierte Acrylamide sowie die wasserlöslichen Alkali-, Erdalkali- und Ammoniumsalze derselben.
ausgewählt ist.

6. Verfahren zur Herstellung von Polymerisaten nach Anspruch 4, **dadurch gekennzeichnet, daß** die Monomerlösung mindestens ein nichtionisches Monomer der allgemeinen Formel I 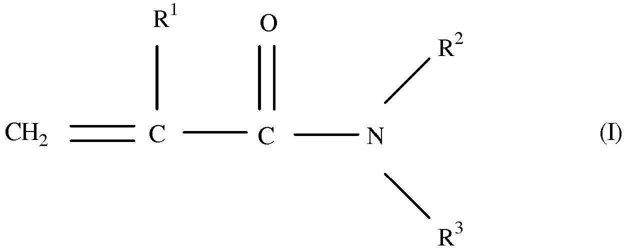 in der
R¹ für einen Wasserstoff oder einen Methylrest, und
R² und R³ unabhängig voneinander, für Wasserstoff, für einen Alkyl- oder Hydroxyalkyl-Rest mit 1 bis 5 C-Atomen
R² oder R³ für eine OH-Gruppe
stehen, umfaßt.

7. Verfahren zur Herstellung von Polymerisaten nach Anspruch 4, **dadurch gekennzeichnet, daß** die Monomerlösung mindestens ein kationisches Monomeres der allgemeinen Formel (II) 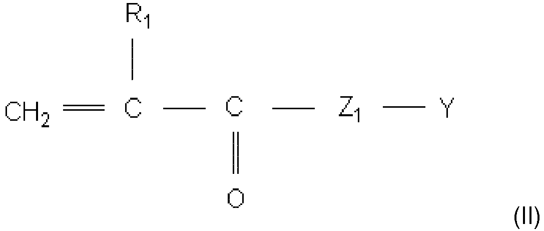 in der
R¹ für Wasserstoff oder einen Methylrest,
Z₁ für O, NH oder NR₄ mit R₄ für einen Alkylrest mit 1 bis 4 C-Atomen,
Y für eine der Gruppen 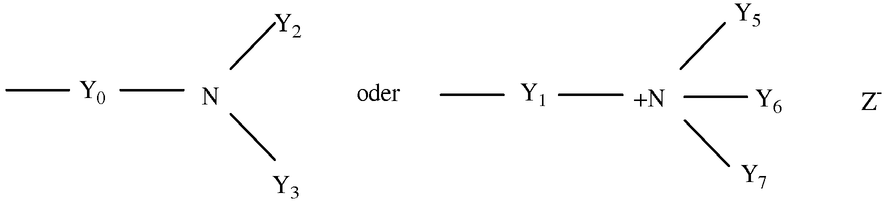
stehen,
wobei
Y₀ und Y₁ für einen Alkylenrest oder Hydroxyalkylenrest mit 2 bis 6 C-Atomen,
Y₂, Y₃, Y₄, Y₅, Y₆, Y₇, unabhängig voneinander, jeweils für einen Alkylrest mit 1 bis 6 C-Atomen,
Z⁻ für Halogen, Acetat, SO₄CH₃⁻ stehen,
umfaßt.

8. Verfahren zur Herstellung von Polymerisaten nach Anspruch 4, **dadurch gekennzeichnet, daß** die Monomerlösung mindestens ein amphiphiles Monomeres der allgemeinen Formel (III) oder (IV) 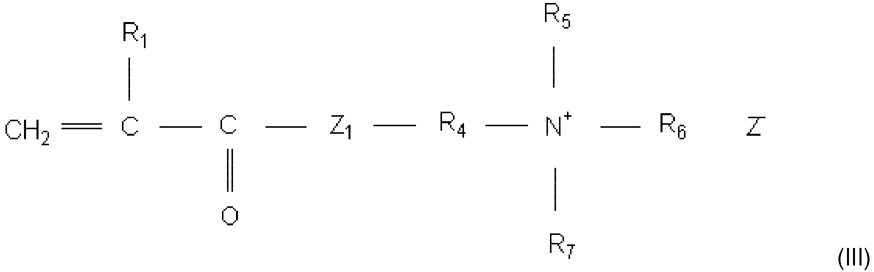 wobei
Z₁ für O, NH, NR₄ mit R₄ für Alkyl mit 1 bis 4 Kohlenstoffatomen,
R₁ für Wasserstoff oder einen Methylrest,
R₄ für Alkylen mit 1 bis 6 Kohlenstoffatomen,
R₅ und R₆ unabhängig voneinander, für einen Alkylrest mit 1 bis 6 Kohlenstoffatomen,
R₇ für einen Alkyl-, Aryl- und/oder Aralkyl-Rest mit 8 bis 32 Kohlenstoffatomen und
Z⁻ für Halogen, Pseudohalogen, SO₄CH3⁻ oder Acetat stehen oder 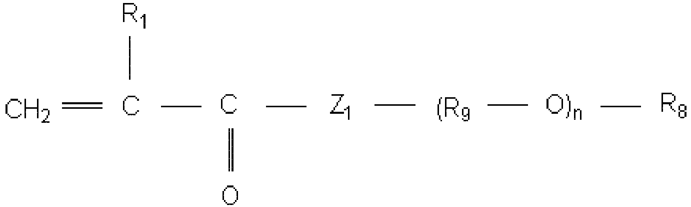 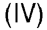
wobei
Z₁ für O, NH, NR₄ mit R₄ für Alkyl mit 1 bis 4 Kohlenstoffatomen,
R₁ für Wasserstoff oder einen Methylrest,
R₈ für Wasserstoff, einen Alkyl-, Aryl- und/oder Aralkyl-Rest mit 8 bis 32 Kohlenstoffatomen,
R₉ für einen Alkylenrest mit 2 bis 6 Kohlenstoffatomen
n für eine ganze Zahl von 1 und 50
stehen, umfaßt.

9. Verfahren zur Herstellung von Polymerisaten nach einem der Ansprüche 1 bis 4, **dadurch gekennzeichnet, daß** die Zusammensetzung der Monomerlösung zur Herstellung des Polymerisate zu 0 bis 100 Gew.-% aus anionischen Monomeren, bezogen auf die Gesamtmenge an Monomeren, besteht.

10. Verfahren zur Herstellung von Polymerisaten nach einem der Ansprüche 1 bis 9, **dadurch gekennzeichnet, daß** die Polymerisation auf einem endlosen Band durchgeführt wird.

11. Verfahren zur Herstellung eines Polymerisates nach einem der Ansprüche 1 bis 10, wobei es sich bei dem Polymerisat um ein Polyacrylamid handelt.

12. Verfahren zur Herstellung von Polymerisaten nach einem der Ansprüche 1 bis 11, **dadurch gekennzeichnet, daß** die Polymerisation adiabatisch durchgeführt wird.

## Claims

1. Process for preparing polymers by photopolymerization, in which a reaction is initiated with UV light and in which irradiation with this UV light is maintained over the entire polymerization reaction in order to assure the complete breakdown of a photoinitiator, in which a monomer solution undergoing polymerization is treated with electromagnetic radiation no earlier than after attainment of the maximum temperature of the polymerization, then the resultant gel is comminuted and dried,
**characterized in that**
the electromagnetic radiation has a greater wavelength than the UV light used to break down the photoinitiator,
and **in that** the electromagnetic radiation is IR radiation.

2. Process for preparing polymers according to Claim 1, in which the monomer solution is polymerized up to a conversion of the monomers of 95 per cent.

3. Process for preparing polymers according to Claim 1, in which the monomer solution is polymerized up to a conversion of the monomers of 99 per cent.

4. Process for preparing polymers according to any of Claims 1 to 3, **characterized in that** the monomer solution comprises anionic, nonionic, cationic and/or amphiphilic ethylenically unsaturated cationic monomers.

5. Process for preparing polymers according to Claim 4, **characterized in that** the monomer solution comprises at least one anionic monomer that is selected from the groups of
a.) olefinically unsaturated carboxylic acids and carboxylic anhydrides, especially acrylic acid, methacrylic acid, itaconic acid, crotonic acid, glutaconic acid, maleic acid, maleic anhydride, fumaric acid, and the water-soluble alkali metal, alkaline earth metal and ammonium salts thereof,
b.) olefinically unsaturated sulfonic acids, especially aliphatic and/or aromatic vinylsulfonic acids, for example vinylsulfonic acid, allylsulfonic acid, styrenesulfonic acid, acryloyl- and methacryloylsulfonic acids, especially sulfoethyl acrylate, sulfoethyl methacrylate, sulfopropyl acrylate, sulfopropyl methacrylate, 2-hydroxy-3-methacryloyloxypropyl-sulfonic acid and 2-acrylamido-2-methylpropanesulfonic acid and the water-soluble alkali metal, alkaline earth metal and ammonium salts thereof,
c.) olefinically unsaturated phosphonic acid, especially vinyl- and allylphosphonic acid and the water-soluble alkali metal, alkaline earth metal and ammonium salts thereof,
d.) sulfomethylated and/or phosphonomethylated acrylamide and the water-soluble alkali metal, alkaline earth metal and ammonium salts thereof.

6. Process for preparing polymers according to Claim 4, **characterized in that** the monomer solution comprises at least one anionic monomer of the general formula I 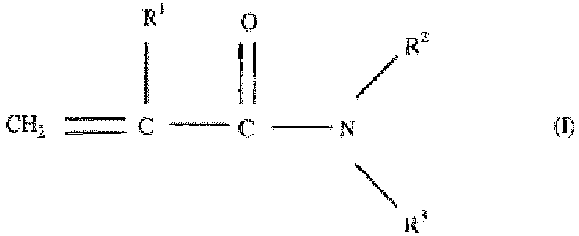 in which
R¹ is a hydrogen or a methyl radical, and
R² and R³ are independently hydrogen or an alkyl or a hydroxyalkyl radical having 1 to 5 carbon atoms,
R² or R³ is an OH group.

7. Process for preparing polymers according to Claim 4, **characterized in that** the monomer solution comprises at least one cationic monomer of the general formula (II) 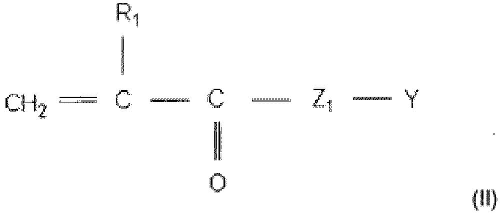 in which
R¹ is hydrogen or a methyl radical,
Z₁ is 0, NH or an NR₄ with R₄ representing an alkyl radical having 1 to 4 carbon atoms,
Y is one of the groups 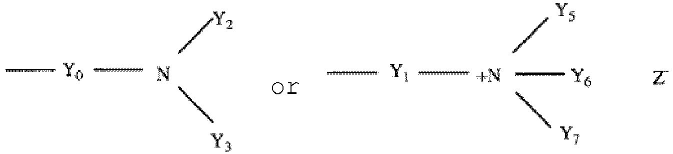
where
Y₀ and Y₁ are an alkylene radical or hydroxyalkylene radical having 2 to 6 carbon atoms,
Y₂, Y₃, Y₄, Y₅, Y₆, Y₇ are each independently an alkyl radical having 1 to 6 carbon atoms,
Z⁻ is halogen, acetate, SO₄CH₃⁻.

8. Process for preparing polymers according to Claim 4, **characterized in that** the monomer solution comprises at least one amphiphilic monomer of the general formula (III) or (IV) 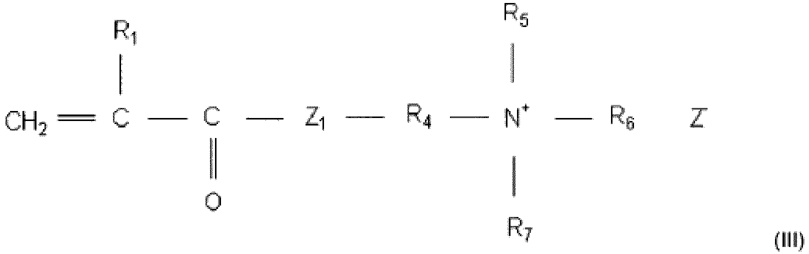 where
Z₁ is O, NH, NR₄ with R₄ representing alkyl radical having 1 to 4 carbon atoms,
R₁ is hydrogen or a methyl radical,
R₄ is alkylene having 1 to 6 carbon atoms,
R₅ and R₆ are independently an alkyl radical having 1 to 6 carbon atoms,
R₇ is an alkyl, aryl and/or aralkyl radical having 8 to 32 carbon atoms and
Z⁻ is halogen, pseudohalogen, SO₄CH₃⁻ or acetate or 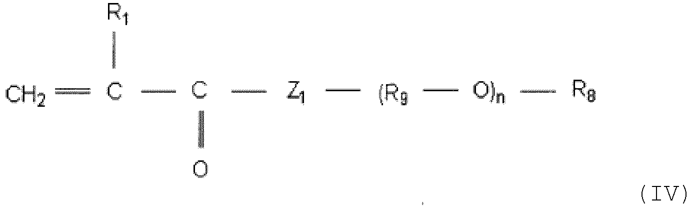
where
Z₁ is 0, NH, NR₄ with R₄ representing alkyl radical having 1 to 4 carbon atoms,
R₁ is hydrogen or a methyl radical,
R₈ is hydrogen, an alkyl, aryl and/or aralkyl radical having 8 to 32 carbon atoms,
R₉ is an alkylene radical having 2 to 6 carbon atoms,
n is an integer of 1 and 50.

9. Process for preparing polymers according to any of Claims 1 to 4, **characterized in that** the composition of the monomer solution for preparation of the polymers consists to an extent of 0% to 100% by weight of anionic monomers, based on the total amount of monomers.

10. Process for preparing polymers according to any of Claims 1 to 9, **characterized in that** the polymerization is performed on a continuous belt.

11. Process for preparing a polymer according to any of Claims 1 to 10, wherein the polymer is a polyacrylamide.

12. Process for preparing polymers according to any of Claims 1 to 11, **characterized in that** the polymerization is performed adiabatically.

## Revendications

1. Procédé de fabrication de polymères par photopolymérisation, selon lequel une réaction est démarrée avec de la lumière UV et selon lequel une exposition à cette lumière UV est maintenue pendant l'ensemble de la réaction de polymérisation afin d'assurer la décomposition totale d'un photoinitiateur, selon lequel une solution de monomères se trouvant dans la polymérisation est traitée avec un rayonnement électromagnétique au plus tôt après l'atteinte de la température maximale de la polymérisation, puis le gel obtenu est broyé et séché,
**caractérisé en ce que**
le rayonnement électromagnétique présente une longueur d'onde plus grande que la lumière UV utilisée pour la décomposition du photoinitiateur,
et **en ce que** le rayonnement électromagnétique consiste en un rayonnement IR.

2. Procédé de fabrication de polymères selon la revendication 1, selon lequel la solution de monomères est polymérisée jusqu'à une conversion des monomères de 95 pour cent.

3. Procédé de fabrication de polymères selon la revendication 1, selon lequel la solution de monomères est polymérisée jusqu'à une conversion des monomères de 99 pour cent.

4. Procédé de fabrication de polymères selon l'une quelconque des revendications 1 à 3, **caractérisé en ce que** la solution de monomères comprend des monomères anioniques, non ioniques, cationiques et/ou amphiphiles éthyléniquement insaturés cationiques.

5. Procédé de fabrication de polymères selon la revendication 4, **caractérisé en ce que** la solution de monomères comprend au moins un monomère anionique, qui est choisi dans les groupes suivants :
a) les acides carboxyliques et anhydrides d'acides carboxyliques oléfiniquement insaturés, notamment l'acide acrylique, l'acide méthacrylique, l'acide itaconique, l'acide crotonique, l'acide glutaconique, l'acide maléique, l'anhydride de l'acide maléique, l'acide fumarique, ainsi que les sels alcalins, alcalino-terreux et d'ammonium solubles dans l'eau de ceux-ci,
b) les acides sulfoniques oléfiniquement insaturés, notamment les acides vinylsulfoniques aliphatiques et/ou aromatiques, par exemple l'acide vinylsulfonique, l'acide allylsulfonique, l'acide styrène-sulfonique, l'acide acryl- et méthacrylsulfonique, notamment l'acrylate de sulfoéthyle, le méthacrylate de sulfoéthyle, l'acrylate de sulfopropyle, le méthacrylate de sulfopropyle, l'acide 2-hydroxy-3-méthacryloxypropylsulfonique et l'acide 2-acrylamido-2-méthylpropane-sulfonique, ainsi que les sels alcalins, alcalino-terreux et d'ammonium solubles dans l'eau de ceux-ci,
c) les acides phosphoniques oléfiniquement insaturés, notamment p. ex. l'acide vinyl- et allylphosphonique, ainsi que les sels alcalins, alcalino-terreux et d'ammonium solubles dans l'eau de ceux-ci,
d) les acrylamides sulfométhylés et/ou phosphonométhylés, ainsi que les sels alcalins, alcalino-terreux et d'ammonium solubles dans l'eau de ceux-ci.

6. Procédé de fabrication de polymères selon la revendication 4, **caractérisé en ce que** la solution de monomères comprend au moins un monomère non ionique de la formule générale I : 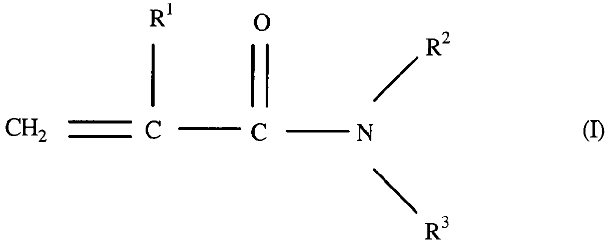 dans laquelle
R¹ représente un hydrogène ou un radical méthyle, et
R² et R³ représentent indépendamment l'un de l'autre un hydrogène, un radical alkyle ou hydroxyalkyle de 1 à 5 atomes C,
R² ou R³ représente un groupe OH.

7. Procédé de fabrication de polymères selon la revendication 4, **caractérisé en ce que** la solution de monomères comprend au moins un monomère cationique de la formule générale (II) : 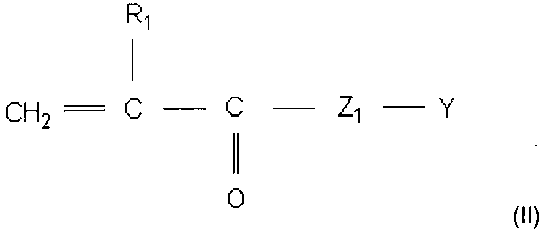 dans laquelle
R¹ représente un hydrogène ou un radical méthyle,
Z₁ représente 0, NH ou NR₄ avec R₄ représentant un radical alkyle de 1 à 4 atomes C,
Y représente un des groupes : 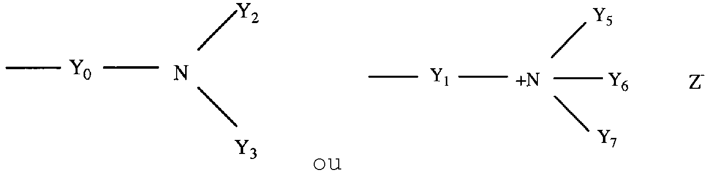 dans lesquels
Y₀ et Y₁ représentent un radical alkylène ou un radical hydroxyalkylène de 2 à 6 atomes C,
Y₂, Y₃, Y₄, Y₅, Y₆, Y₇ représentent chacun indépendamment les uns des autres un radical alkyle de 1 à 6 atomes C,
Z⁻ représente un halogène, un acétate, SO₄CH₃⁻.

8. Procédé de fabrication de polymères selon la revendication 4, **caractérisé en ce que** la solution de monomères comprend au moins un monomère amphiphile de la formule générale (III) ou (IV) : 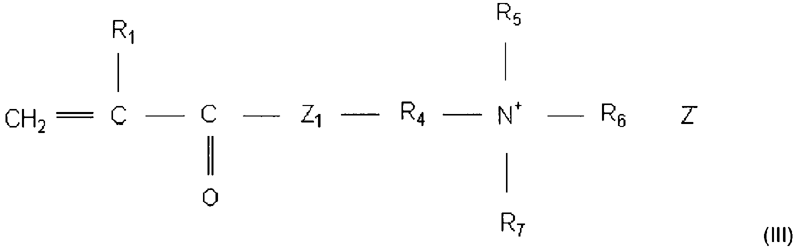 dans laquelle
Z₁ représente 0, NH, NR₄ avec R₄ représentant un alkyle de 1 à 4 atomes de carbone,
R₁ représente un hydrogène ou un radical méthyle,
R₄ représente un alkylène de 1 à 6 atomes de carbone,
R₅ et R₆ représentent indépendamment l'un de l'autre un radical alkyle de 1 à 6 atomes de carbone,
R₇ représente un radical alkyle, aryle et/ou aralkyle de 8 à 32 atomes de carbone, et
Z⁻ représente un halogène, un pseudohalogène, SO₄CH₃⁻ ou un acétate, ou 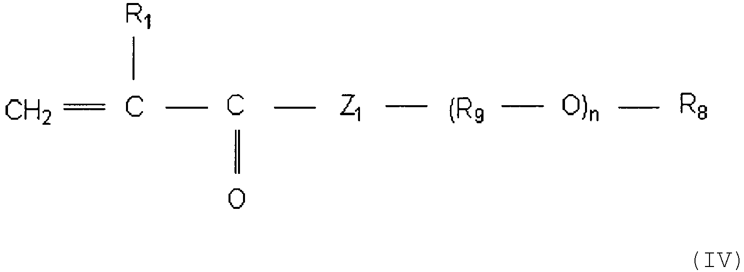 dans laquelle
Z₁ représente 0, NH, NR₄ avec R₄ représentant un alkyle de 1 à 4 atomes de carbone,
R₁ représente un hydrogène ou un radical méthyle,
R₈ représente un hydrogène, un radical alkyle, aryle et/ou aralkyle de 8 à 32 atomes de carbone,
R₉ représente un radical alkylène de 2 à 6 atomes de carbone,
n représente un nombre entier de 1 et 50.

9. Procédé de fabrication de polymères selon l'une quelconque des revendications 1 à 4, **caractérisé en ce que** la composition de la solution de monomères pour la fabrication des polymères est constituée à hauteur de 0 à 100 % en poids par des monomères anioniques, par rapport à la quantité totale de monomères.

10. Procédé de fabrication de polymères selon l'une quelconque des revendications 1 à 9, **caractérisé en ce que** la polymérisation est réalisée sur une bande sans fin.

11. Procédé de fabrication d'un polymère selon l'une quelconque des revendications 1 à 10, **caractérisé en ce que** le polymère consiste en un polyacrylamide.

12. Procédé de fabrication de polymères selon l'une quelconque des revendications 1 à 11, **caractérisé en ce que** la polymérisation est réalisée de manière adiabatique.
